# EUROPEAN PATENT APPLICATION

(11) **EP 4 706 499 A1**
(43) Date of publication of application: **11.03.2026**
(21) Application number: 24198664.5
(22) Date of filing: 05.09.2024
(51) Int. Cl.: A61B 3/10, A61B 3/12, A61B 3/14

(54) **CONTROL OF ILLUMINATION FROM A SCANNING OPHTHALMOSCOPE**

(71) Applicant: Optos plc, Dunfermline, Scotland KY11 8GR (GB)
(72) Inventor: SWAN, Derek, Dunfermline, Scotland, KY11 8GR (GB); ANDERSON, Alan, Dunfermline, Scotland, KY11 8GR (GB); MUYO, Gonzalo, Dunfermline, Scotland, KY11 8GR (GB)
(74) Representative: Hoffmann Eitle

(57) **Abstract**

An ophthalmic imaging instrument for imaging an eye of a subject, comprising: a light source arranged to emit a beam of light for imaging the eye; an optical system comprising a rotatable mirror and a drive mechanism arranged to drive the rotatable mirror to undergo a predetermined rotational motion to scan the beam of light across the eye; a rotary encoder coupled to the rotatable mirror so as to generate a signal indicative of a rotational motion of the rotatable mirror; and a processor arranged to detect when the rotational motion of the rotatable mirror indicated by the signal has deviated from the predetermined rotational motion and, in response to detecting that the rotational motion of the rotatable mirror has deviated from the predetermined rotational motion, generate a control signal to reduce an illumination of the eye by the beam of light.

## Description

### [Field]

Example aspects herein generally relate to the field of ophthalmic imaging scanners and, more particularly, to safety mechanisms employed in ophthalmic imaging scanners to limit exposure of a subject's eye to illumination therefrom.

### [Background]

Ophthalmic imaging scanners of different kinds, including scanning laser ophthalmoscopes (SLOs) of various imaging modalities and optical coherence tomography (OCT) scanners, are widely used to assess the health of patients' eyes. With patient safety being paramount, measures are usually taken to ensure that an ophthalmic imaging scanner cannot expose a patient's eye to a level of radiation that would exceed the maximum permissible exposure (MPE) or a desired fraction thereof. These measures typically include meticulous tuning and testing of the scanner during commissioning, and providing the scanner with safety mechanisms to monitor aspects of beam delivery, such as its duration and optical power, in real-time during subsequent use, and to ensure that these do not depart from safe norms.

### [Summary]

There is provided, in accordance with a first example aspect herein, an ophthalmic imaging instrument for imaging an eye of a subject, comprising: a light source arranged to emit a beam of light for imaging the eye; an optical system comprising a rotatable mirror and a drive mechanism arranged to drive the rotatable mirror to undergo a predetermined rotational motion to scan the beam of light across the eye; and a rotary encoder coupled to the rotatable mirror so as to generate a signal indicative of a rotational motion of the rotatable mirror. The ophthalmic imaging instrument further comprises a processor arranged to: detect when the rotational motion of the rotatable mirror indicated by the signal has deviated from the predetermined rotational motion; and in response to detecting that the rotational motion of the rotatable mirror has deviated from the predetermined rotational motion, generate a control signal to reduce an illumination of the eye by the beam of light.

In example embodiments, the rotary encoder may comprise: an encoder wheel attached to the rotatable mirror so as to rotate with the rotatable mirror, the encoder wheel having markings for detecting a rotation of the encoder wheel; and a detector arranged to detect the markings on the encoder wheel as the encoder wheel rotates. In this case, the processor may be arranged to: monitor detections of the markings by the detector; and detect, from the monitored detections, when the rotational motion of the rotatable mirror has deviated from the predetermined rotational motion.

In some of these example embodiments, the markings of the encoder wheel are provided at uniformly spaced angular positions about an axis of rotation of the encoder wheel. In these example embodiments, the ophthalmic imaging instrument may be an ultra-widefield ophthalmic imaging instrument for imaging a fundus of the eye, and the optical system further may comprise a curved (e.g. spheroidal) mirror via which the rotatable mirror is arranged to scan the beam of light across the fundus, the curved mirror having an axis of circular symmetry, a first focal point and a second focal point, wherein the rotatable mirror is arranged to scan the beam of light across the curved mirror via the first focal point (for example, by the rotatable mirror being arranged to reflect the beam of light at the first focal point such that the reflected beam propagates to the curved mirror) and has an axis of rotation aligned with the axis of circular symmetry of the curved mirror, and a pupil of the eye is positioned at the second focal point during the imaging of the fundus. Furthermore, the processor may be further arranged to generate a respective indication of a respective scan position of the beam of light on the fundus based on a detection by the detector of each marking of the encoder wheel, and associate the respective indication with respective image data acquired by the ophthalmic imaging instrument at the respective scan position.

In some other of the example embodiments, the predetermined rotational motion is a predetermined oscillatory rotational motion, wherein the rotatable mirror rotates alternatively clockwise and anticlockwise by a predetermined angle, and the markings of the encoder wheel are provided at non-uniformly spaced angular positions about an axis of rotation of the encoder wheel that have angular separations therebetween which decrease towards each marking of the markings which is last detected by the detector before the encoder wheel changes its direction of rotation when rotating with the rotatable mirror alternatively clockwise and anticlockwise by the predetermined angle. In these example embodiments, at least some of the markings of the encoder wheel may be provided at non-uniformly spaced angular positions about the axis of rotation of the encoder wheel such that the detector detects the at least some of the markings at a constant frequency when the encoder wheel rotates alternatively clockwise and anticlockwise with the rotatable mirror when undergoing the predetermined oscillatory rotational motion.

In some other of the example embodiments, the ophthalmic imaging instrument is an ultra-widefield ophthalmic imaging instrument for imaging a fundus of the eye, wherein the optical system further comprises a curved mirror via which the rotatable mirror is arranged to scan the beam of light across the fundus, the curved mirror having a first focal point and a second focal point, wherein the rotatable mirror is arranged to scan the beam of light across the curved mirror via the first focal point (for example, by the rotatable mirror being arranged to reflect the beam of light at the first focal point, such that the reflected beam propagates to the curved mirror), and a pupil of the eye is positioned at the second focal point during the imaging of the fundus, and the curved mirror has a curvature such that an angle of incidence of the beam of light on a pupillary plane of the eye at the second focal point varies non-linearly with an angle of rotation of the rotatable mirror. Furthermore, the markings of the encoder wheel are provided at non-uniformly spaced angular positions about an axis of rotation of the encoder wheel such that, as the beam of light is scanned via the curved mirror, a common change in the angle of incidence occurs as the rotatable mirror rotates between detections by the detector of different adjacent markings of the encoder wheel, and the processor is further arranged to generate a respective indication of a respective scan position of the beam of light on the fundus based on a detection by the detector of each marking of the encoder wheel, and associate the respective indication with respective image data acquired by the ophthalmic imaging instrument at the respective scan position.

In any of the foregoing example embodiments not having a curved (e.g. spherical) mirror with an axis of circular symmetry, the ophthalmic imaging instrument may be an ultra-widefield ophthalmic imaging instrument for imaging a fundus of the eye, wherein the optical system further comprises a curved mirror via which the rotatable mirror is arranged to scan the beam of light across the fundus, the curved mirror having a first focal point and a second focal point, wherein the rotatable mirror is arranged to scan the beam of light across the curved mirror via the first focal point (for example, by the rotatable mirror being arranged to reflect the beam of light at the first focal point, such that the reflected beam propagates to the curved mirror), and a pupil of the eye is positioned at the second focal point during the imaging of the fundus, and the curved mirror has a curvature such that an angle of incidence of the beam of light on a pupillary plane of the eye at the second focal point varies non-linearly with an angle of rotation of the rotatable mirror. Furthermore, the rotary encoder may further comprise second markings that are detectable by the detector as the encoder wheel rotates, and the second markings may be provided at non-uniformly spaced angular positions about an axis of rotation of the second markings such that, as the beam of light is scanned via the curved mirror, a common change in the angle of incidence occurs as the rotatable mirror rotates between detections by the detector of different adjacent markings of the second markings. Furthermore, the processor may be further arranged to generate a respective indication of a respective scan position of the beam of light on the fundus based on a detection by the detector of each second marking, and associate the respective indication with respective image data acquired by the ophthalmic imaging instrument at the respective scan position. The second markings may be provided on the encoder wheel. Alternatively, the rotary encoder may further comprise a second encoder wheel attached to the rotatable mirror so as to rotate with the rotatable mirror, the second markings may be provided on the second encoder wheel, and the detector may be arranged to detect the second markings on the second encoder wheel as the second encoder wheel rotates.

In any of the foregoing example embodiments, the processor may be arranged to detect when the rotational motion of the rotatable mirror has deviated from the predetermined rotational motion by repeatedly preforming, for each time interval of a plurality of time intervals, processes of: counting detections by the detector in the time interval; and detecting that the rotational motion of the rotatable mirror has deviated from the predetermined rotational motion when the counted detections in the time interval are fewer than a threshold number of detections.

In any of the foregoing example embodiments, the drive mechanism may comprise a galvanometer having a first drive shaft, and a second drive shaft which is attached to the rotatable mirror and rotationally coupled to the first drive shaft such that the rotatable mirror undergoes the predetermined rotational motion when driven by the galvanometer, wherein the encoder wheel is mounted on the second drive shaft to rotate with the rotatable mirror.

The ophthalmic imaging instrument according to the first example aspect of any of the example embodiments set out above may further comprise at least one of: a power supply arranged to supply power to the light source, wherein the generated control signal is arranged to control the power supply to reduce power supplied to the light source; or a shutter operable to prevent the beam of light from reaching the eye, wherein the generated control signal is arranged to control the shutter to prevent the beam of light from reaching the eye.

There is provided, in accordance with a second example aspect herein, a computer-implemented method of generating a control signal for controlling an ophthalmic imaging instrument for imaging an eye of a subject, the ophthalmic imaging instrument comprising: a light source arranged to emit a beam of light for imaging the eye; an optical system comprising a rotatable mirror and a drive mechanism arranged to drive the rotatable mirror to undergo a predetermined rotational motion to scan the beam of light across the eye; and a rotary encoder coupled to the rotatable mirror so as to generate a signal indicative of a rotational motion of the rotatable mirror. The method comprises: detecting when the rotational motion of the rotatable mirror indicated by the signal has deviated from the predetermined rotational motion; and in response to detecting that the rotational motion of the rotatable mirror has deviated from the predetermined rotational motion, generating a control signal to reduce an illumination of the eye by the beam of light.

In some example embodiments, the ophthalmic imaging instrument may further comprise a shutter operable to prevent the beam of light from reaching the eye, and the method may further comprise using generated control signal to control the shutter to prevent the beam of light from reaching the eye. In these or other example embodiments, the ophthalmic imaging instrument may further comprise a power supply arranged to supply power to the light source, and the method may further comprise controlling the power supply using the generated control signal to reduce power supplied to the light source.

There is also provided, in accordance with a third example aspect herein, a computer program comprising computer-readable instructions that, when executed by at least one processor, cause the at least one processor to perform the method of the second example aspect or any of its embodiments set out above. The computer program may be stored on a non-transitory computer-readable storage medium (such as a computer hard disk or a CD, for example) or carried by a computer-readable signal.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an ultra-widefield ophthalmic imaging instrument according to a first example embodiment herein.
Figure 2 is a schematic side view of the rotary encoder of the first example embodiment herein, which shows some further details of the drive mechanism of the first example embodiment herein.
Figure 3A is a schematic plan view of an encoder wheel of the rotary encoder in the first example embodiment herein, which has uniformly spaced markings.
Figure 3B is a schematic plan view of an alternative encoder wheel, which is employed in a variant of the first example embodiment herein and has non-uniformly spaced markings.
Figure 3C is a schematic plan view of an encoder wheel of the rotary encoder 38 in the second example embodiment herein, which has non-uniformly spaced markings for faster failure detection.
Figure 3D is a schematic plan view of an encoder wheel of a first variant of a second example embodiment herein, which has both the markings of the encoder wheel of Figure 3A and the markings of the encoder wheel of Figure 3C.
Figure 3E is a schematic plan view of an encoder wheel of a second variant of the second example embodiment herein, which has both the markings of the encoder wheel of Figure 3B and the markings of the encoder wheel of Figure 3C.
Figure 4 is a schematic illustration of an example of an optical system forming part of the ophthalmic imaging instrument of the first example embodiment herein.
Figure 5 is a flow diagram illustrating a method by which a processor generates a control signal for controlling the ophthalmic imaging instrument of an example embodiment.
Figure 6 is a schematic illustration of a programmable signal processing hardware, which may be configured to perform the at least some of the processes of the methods described herein.
Figure 7 is a schematic illustration of an ultra-widefield ophthalmic imaging instrument according to the second example embodiment herein.
Figure 8 is a schematic illustration of an example of an optical system forming part of the ophthalmic imaging instrument of the second example embodiment herein.
Figure 9 is a schematic illustration of an ultra-widefield ophthalmic imaging instrument according to a variant of the second example embodiment herein, which comprises two curved mirrors having a shared focal point, wherein the rotating mirror is provided at the remaining focal point of one of the curved mirrors to scan line-field illumination across a fundus via a pupil provided at the remaining focal point of the other curved mirror.

### [Detailed Description of Example Embodiments]

With patient safety in mind, the inventors have devised, in accordance with example embodiments herein, an ophthalmic imaging instrument having a rotatable mirror which scans a beam of light across the eye when driven to undergo a predetermined rotational motion during image acquisition, and a mechanism for reducing an illumination of the eye by the beam when it detects that the mirror has deviated from its predetermined rotational motion. More particularly, the ophthalmic imaging instrument further comprises a rotary encoder coupled to the mirror so as to generate a signal indicative of a rotational motion of the mirror, and a processor arranged to detect when the (actual) rotational motion of the mirror indicated by the signal has deviated from the (expected) predetermined rotational motion. The processor is arranged to generate a control signal to stop the illumination of the eye by the beam or to decrease the optical power of the beam to a prescribed safe level, for example, in response to detecting that the rotational motion of the mirror has deviated from the predetermined rotational motion. The illumination of the eye by the beam of light may thus be reduced in case the mirror is detected to deviate from its predetermined rotational motion, which may carry the risk of the beam moving more slowly or being stationary on the eye and thus causing damage to the eye. Example embodiments herein will now be described in detail with reference to accompanying drawings.

### [Example Embodiment 1]

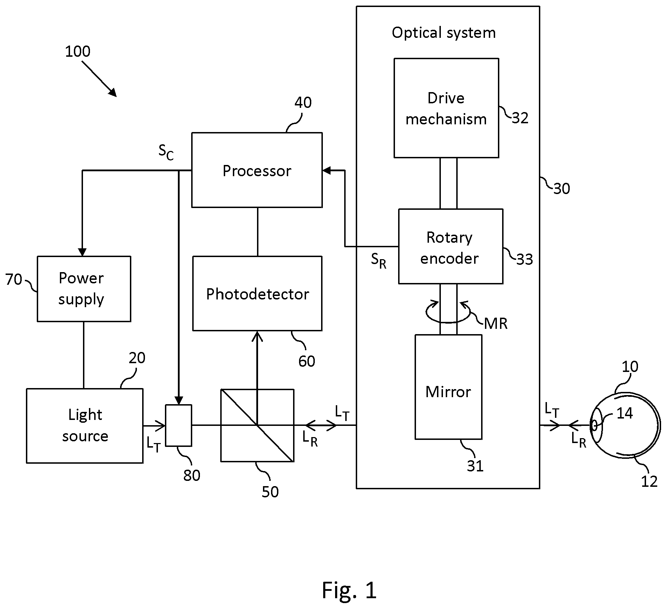

Figure 1 is a schematic illustration of an ophthalmic imaging instrument 100 according to the first example embodiment, which is operable to image a portion of an eye 10 of a subject. The portion imaged may, as in the present example embodiment, comprise a region of an ocular fundus 12 of the eye 10. The ocular fundus 12 is the inner lining of the eye 10 opposite the lens, and comprises the retina, macula, optic disc, fovea, choroid, and blood vessels. The ophthalmic imaging instrument 100 may additionally or alternatively be operable to image another portion of the eye 10, such as at least a part of the anterior segment of the eye 10.

The ophthalmic imaging instrument 100 may be a widefield (WF) ophthalmic imaging instrument, which is operable to acquire a WF image of the fundus 12. A WF image of the fundus 12 is defined as a single-capture image, centred on the fovea of the eye 10, which captures retinal anatomic features in the mid-periphery that are posterior to the vortex vein ampulla, in all four quadrants (i.e. superior, inferior, nasal and temporal) of the eye 10. A WF ophthalmic imaging instrument can thus acquire a single-capture image which covers a region of the retina extending from the fovea up to the posterior edge of vortex vein ampulla, and is defined to have a field of view (FoV) between 60 and 100 degrees. Here, the FoV is expressed in terms of the eye-angle, which is the angle subtended by the imaged retinal region at the spherical centre of the eye 10, i.e. the point of intersection between the vertical diameter of the eyeball and the visual axis.

The ophthalmic imaging instrument 100 may, as in the present example embodiment, be provided in form of an ultra-widefield (UWF) ophthalmic imaging instrument, which has a larger FoV than a WF instrument and is operable to acquire an UWF image of the ocular fundus 12 covering a larger proportion of the ocular fundus 12. An UWF image of the ocular fundus 12 is defined as a single-capture image, centred on the fovea of the eye 10, which captures retinal anatomic features in the far periphery that are anterior to the vortex vein ampulla in all four quadrants. An UWF ophthalmic imaging instrument can thus acquire an UWF image being a single-capture image which covers a region of the retina extending from the fovea to the anterior edge of vortex vein ampulla and beyond to pars plana, and is defined to have a FoV (expressed in terms of the eye-angle) between 110 degrees and 220 degrees. By way of an example, the Optos Daytona^{™} is capable of UWF images (so -called Optomap^{™} images) covering up to 200 degrees of the fundus (or approximately 82% of the retina) in a single capture.

It should be noted that the techniques described herein are not limited in their applicability to WF and UWF ophthalmic imaging instruments but are also applicable to ophthalmic imaging instruments having a smaller FoV, and may benefit any scanning ophthalmic imaging instrument which scans light across a portion of the eye 10 to image the portion, particularly point-scan instruments that scan a beam of light across the eye 10 in a raster or other scan pattern.

In the present example embodiment, the ophthalmic imaging instrument 100 comprises an UWF combined scanning laser ophthalmoscope (SLO) and optical coherence tomography (OCT) instrument, which is arranged to acquire OCT images of the ocular fundus 12 using well-known interferometric imaging techniques, as well as fundus images of one or more additional modalities. Examples of such additional imaging modalities include pseudo-colour imaging, fundus autofluorescence (FAF), fluorescein angiography (FA), and indocyanine green angiography (ICGA). By way of an example, the Optos Monaco^{™} is a combined SLO-OCT system capable of acquiring green or red (or combined green and red) laser views, and green laser autofluorescence views, as well as OCT views. However, the ophthalmic imaging instrument 100 need not be a combined SLO-OCT system, and its available imaging modalities may be limited to OCT only, or to one or more of the aforementioned (or other) SLO imaging modalities.

As illustrated schematically in Figure 1, the ophthalmic imaging instrument 100 comprises a light source 20 arranged to emit at least one beam of light L_{T} for imaging the eye 10, and an optical system 30 arranged to scan the at least one beam of light L_{T} across the eye 10, and collect and guide (using one or more optical components, such as optical fibres, lenses and mirrors) return light L_{R} from the region of the eye 10 illuminated by the beam of light L_{T}. The ophthalmic imaging instrument 100 further comprises a processor (or controller) 40, and may, as in the present example embodiment, also include a beam splitter 50 and a photodetector 60, wherein the beam splitter 50 is arranged to split off a portion of the return light L_{R} and direct the split-off portion of the return light L_{R} to the photodetector 60. The image of the eye 10 is acquired by the ophthalmic imaging instrument 100 using the output of the photodetector 60. The ophthalmic imaging instrument 100 further comprises a power supply 70 arranged to supply power to the light source 20, and may also have a shutter 80 which is operable to prevent the beam of light L_{T} emitted by the light source 20 from reaching the eye 10. The shutter 80 may be located between the light source 20 and the beam splitter 50, as in the example of Figure 1, or anywhere else in the optical path of the beam of light L_{T} between the light source 20 and the eye 10.

The optical system 30 comprises a rotatable mirror 31, and a drive mechanism 32 arranged to drive the rotatable mirror 31 to undergo a predetermined rotational motion MR to scan the beam of light L_{T} across the eye 10. The predetermined rotational motion MR may, as in the present example embodiment, comprise a predetermined oscillatory rotational motion, wherein the rotatable mirror 31 rotates alternatively clockwise and anticlockwise by a predetermined angle (or, in other words, wherein the rotatable mirror 31 repeatedly rotates across an angular range defined about the mirror's axis of rotation, reversing its direction of rotation at each end of the angular range). The predetermined rotational motion MR is not so limited, however, and may alternatively comprise a rotation at a predetermined constant angular velocity in case of the rotatable mirror 31 forming part of a polygon scanner, for example.

The ophthalmic imaging instrument 100 further comprises a rotary encoder 33, which is coupled to the rotatable mirror 31 so as to generate a signal S_{R} indicative of a rotational motion of the rotatable mirror 31 when the rotatable mirror 31 rotates about its axis of rotation. As will be described in more detail below, the processor 40 is arranged to detect when the rotational motion of the rotatable mirror 31 indicated by the signal S_{R} has deviated from the predetermined rotational motion MR and, in response to detecting the deviation, generate a control signal S_{C} to reduce an illumination of the eye 10 by the beam of light L_{T}. The generated control signal S_{C} may, as in the present example embodiment, control the power supply 70 to reduce power supplied to the light source 20 to a low level which is safe for the eye 10 even when the beam of light L_{T} is incident on the eye 10 whilst not being scanned across the eye 10, or to zero power so that the light source 20 is turned off. Where the ophthalmic imaging instrument 100 is provided with the shutter 80 as described above, the generated control signal Sc may control the shutter 80 to close, thereby preventing the beam of light L_{T} from reaching the eye 10. For improved reliability, the processor 40 may respond to the detection of the aforementioned deviation by both controlling the power supply 70 to reduce the power supplied to the light source 20 and controlling the shutter 80 to close.

The light source 20 is, in general, arranged to generate light in one or more ranges of wavelength that are suitable for imaging the ocular fundus 12 (or other part of the eye 10, as the case may be), for example in the visible spectrum (e.g. red and green light) and/or the near-infrared spectrum. The light source 20 may, for example, comprise one or more laser diodes or one or more super-luminescent diodes (or a combination of one or more laser diodes and one or more super-luminescent diodes), and may also have one or more optical components, such as collimators, apertures and lenses, which are arranged to generate one or more light beams. The optical system 30 (discussed further below) may be arranged to illuminate a region of the ocular fundus 12 with a (flying) spot of light or a line of light (in case of the ophthalmic imaging instrument 100 being a line-field system) generated using a cylindrical lens, a slit or other well-known components or optical assemblies for generating line-field illumination.

In example embodiments like the present, wherein the ophthalmic imaging instrument 100 is operable in an OCT imaging mode, the light source 20 may comprise a swept light source (in case of the ophthalmic imaging instrument 100 being a swept-source OCT (SS-OCT) system) or a broadband source (in case of the ophthalmic imaging instrument 100 being a spectral-domain OCT (SD-OCT) system).

The form of the photodetector 60 is not limited and it may, for example, comprise a balanced photodetector arrangement comprising two reverse-biased photodiodes whose output photocurrents are subtracted from one another, the subtracted current signal being converted to a voltage detection signal by a transimpedance amplifier. In example embodiments like the present, where the ophthalmic imaging instrument 100 features an OCT imaging modality, the photodetector 60 may comprise a spectrometer (where a SD-OCT set-up is used) or a photodiode detector (where a SS-OCT set-up is used).

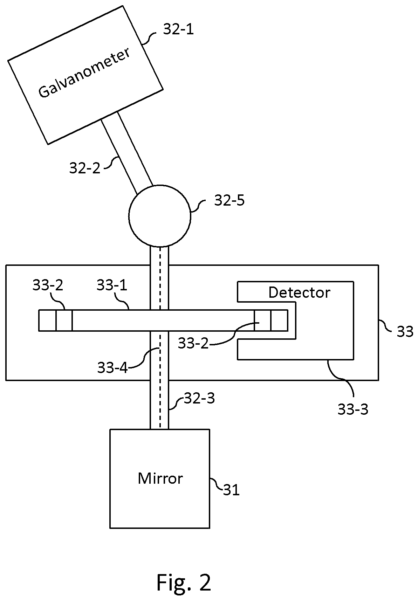

Figure 2 is a schematic illustration showing further details of the drive mechanism 32 and the rotary encoder 33 of Figure 1. The rotary encoder 33 may, as illustrated schematically in Figure 2, comprise an encoder wheel (or encoder disc) 33-1, which is attached to the rotatable mirror 31 so as to rotate as the rotatable mirror 31 rotates, and a detector 33-3 arranged to detect rotation of the encoder wheel 33-1. The encoder wheel 33-1 may be formed of any rigid material, such as plastic, glass or metal, and has markings 33-2 that can be detected by the detector 33-3 as they rotate past the detector 33-3. In this case, the processor 40 is arranged to monitor detections of the markings 33-2 by the detector 33-3 and detect, from the monitored detections, when the rotational motion of the rotatable mirror 31 has deviated from the predetermined rotational motion MR. Examples of how the processor 40 may be configured to detect this deviation are described below.

Where the rotary encoder 33 takes the form of an optical rotary encoder, as in the present example embodiment, the markings 33-2 may take the form of perforations (e.g. holes and/or slots) in the encoder wheel 33-1 that are distributed circumferentially (azimuthally) about an axis of rotation 33-4 of the encoder wheel 33-1. In this case, the detector 33-3 may comprise a light source (not shown), such as a light-emitting diode (LED), which is arranged to illuminate a first side of the encoder wheel 33-1, and a photosensor (not shown), such as a photodiode, which is arranged on the opposite side of the encoder wheel 33-1 to detect light from the light source of the detector 33-3 which has been transmitted through one or more of the perforations in the encoder wheel 33-1.

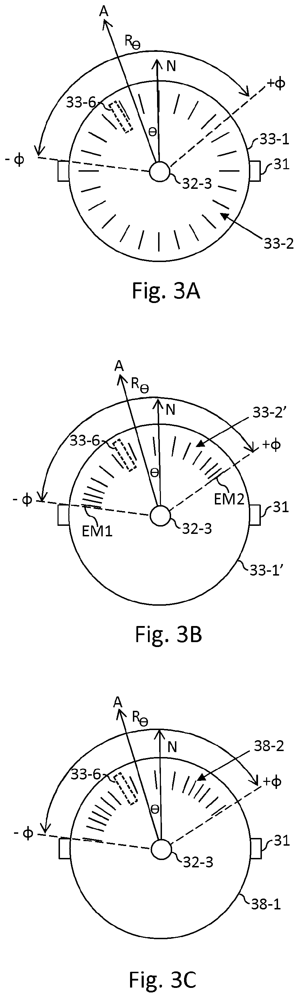

Figure 3A is a schematic illustration of the encoder wheel 33-1 of the present example embodiment. In this example, the markings 33-2 of the encoder wheel 33-1 are provided at regularly spaced angular positions about the axis of rotation 33-4 of the encoder wheel 33-1. When the mirror 31 is driven by the drive mechanism 32 to rotate alternatively clockwise and anticlockwise by the predetermined angle, in accordance with the predetermined oscillatory rotational motion, the encoder wheel 33-1 rotationally coupled by a coupling 32-5 thereto likewise rotates alternatively clockwise and anticlockwise by the predetermined angle. In the example of Figure 3A, this angle is 2ϕ, and corresponds to an angular range over which an angle θ varies during a cycle (or oscillation) of the predetermined oscillatory rotational motion during which the rotational mirror 31 rotates from one extreme of the oscillatory rotation (where θ = - ϕ) to the next (where θ = + ϕ), changing the direction of rotation at each extreme. Here, θ is the angle between a direction N normal to the plane of the (planar) rotatable mirror 31 and a reference axis A, with which the direction N is aligned in the middle of the cycle (where θ = 0). Figure 3A also shows the position of a reading region 33-6 of the detector 33-3, in which the detector 33-3 can detect the presence of a marking. The position of the reading region 33-6 does not change as the encoder wheel 33-1 rotates.

An optical rotary encoder of the general form described above may take many different forms. For example, it may be a masked optical rotary encoder or a phased-array optical rotary encoder. Furthermore, the rotary encoder 33 need not rely on the transmission of light through the encoder wheel, and may take the form of a reflective optical encoder, wherein the light source and photosensor are provided on the same side of the encoder wheel, and the markings are reflective regions provided on an otherwise non-reflective encoder wheel. Furthermore, the rotary encoder 33 need not be an optical rotary encoder, and may instead take the form of a mechanical rotary encoder or a magnetic rotary encoder, for example. Regardless of the type of rotary encoder technology used, the rotary encoder 33 may be an absolute rotary encoder capable of reporting both changes in, and absolute values of, the encoder wheel position, or may be an incremental (or step) encoder capable of reporting changes in the encoder wheel position only. Where the rotary encoder 33 is an absolute rotary encoder, the markings (regardless of their physical form) may be distributed on the encoder wheel (whether at regularly or irregularly spaced angular positions, as described below) so as to encode absolute values of the encoder wheel position using simple binary encoding or, more preferably, a type of Gray code to reduce encoder communication errors, for example.

Measuring the rotation of the rotatable mirror 31 using the output of a rotary encoder, rather than a digitised output of an analog voltage that is output by the drive circuit of many galvanometer scanners to provide an indication of its rotational position, may be advantageous in that the signal-to-noise in the rotary encoder's output signal will normally be independent of the degree of galvanometer deflection/rotation. The deviations from the predetermined rotational motion MR may therefore be detected more reliably.

The optical system 30 of the present example embodiment is arranged to perform a two-dimensional point-scan of the light L_{T} from the light source 20 across the region of the ocular fundus 12 that is illuminated by the point-scan, and to collect light from the illuminated region during the point-scan.

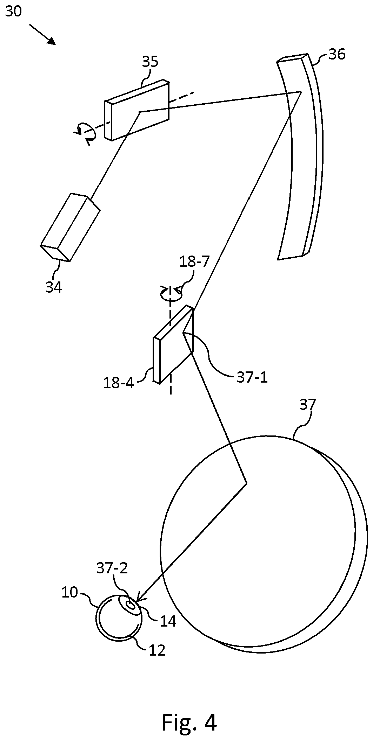

An example of such an optical system is illustrated in Figure 4. The optical system 30 may, as in the present example embodiment, comprise a curved mirror having an axis of circular symmetry, for example in the form of a spheroidal mirror 37, i.e. a mirror whose (open) reflective surface has the curvature of a spheroid. The optical system 30 is arranged to perform the two-dimensional point scan by the rotatable mirror 31 scanning the beam of light L_{T} across the ocular fundus 12 via the spheroidal mirror 37. For clarity, the drive mechanism 32 and rotary encoder 33 of the optical system 30 are not shown in Figure 4. The optical system 30 may further comprise an optical coupler 34, a first scanning element 35, a first curved mirror 36 and the spheroidal mirror 37. The light beam enters the optical system 30 via the optical coupler 34. The light beam is then reflected, in sequence, by the first scanning element 35, the first curved mirror 36, the rotatable mirror 31 and the spheroidal mirror 37, before being incident on the eye 10. Light from the illuminated region of the ocular fundus 12 collected by the optical system 30 follows the same optical path through the optical system 30 as the light beam that had entered the optical system 30 via the optical coupler 34 but does so in reverse order, and exits the optical system 30 via the optical coupler 34.

The two-dimensional point scan is performed by the first scanning element 35 rotating around its rotational axis to scan the light beam in a first direction across the region of the ocular fundus 12, and by the rotatable mirror 31 rotating around the rotational axis 33-4 to scan the light beam in a second direction across the region of the ocular fundus 12 (which second direction may, as in the present example embodiment, be orthogonal to the first direction). Here, rotational axis R of the rotatable mirror 31 is parallel to an axis of circular symmetry of the spheroidal mirror 37. The length of the optical path between the first focal point 37-1 of the spheroidal mirror 37, and points along the trajectory on the spheroidal mirror 27 followed by the beam of light L_{T} incident thereon as the beam of light L_{T} is scanned by rotation of the rotatable mirror 31, is constant. By rotating the first scanning element 35 and the rotatable mirror 31, it is possible to steer the beam of light L_{T} to different locations on the ocular fundus 12. The rotations of the first scanning element 35 and the rotatable mirror 31 may be coordinated by a scanning system controller (not shown) such that the beam of light L_{T} is scanned across the ocular fundus 12 in accordance with a predefined scan pattern.

In the example of Figure 4, the first curved mirror 36 is an ellipsoidal mirror (and referred to as a slit mirror). Each of the first curved mirror 36 and the spheroidal mirror 37 has a respective first focal point and a respective second focal point. The first scanning element 35 is disposed at the first focal point of the first curved mirror 36, and the rotatable mirror 31 is disposed at the second focal point of the first curved mirror 36. The rotatable mirror 31 is also disposed at a first focal point 37-1 of the spheroidal mirror 37, and the eye 10 (more specifically, the pupil 14 of the eye 10 in the present example) is disposed at a second focal point 37-2 of the spheroidal mirror 37.

In the present example embodiment, the first scanning element 35 also takes the form of a galvanometer mirror. However, the first scanning element 35 may be provided in other forms, such as a polygon scanning mirror, a micro-electromechanical system (MEMS) scanning mirror or a resonant scanning mirror, for example.

An optical system 30 of the kind described above with reference to Figure 4 may be used in a variety of imaging modes, such as colour, red-free, AF, ICG and OCT (among others). However, the optical system 30 may alternatively be arranged in other ways, for example to instead perform a line-field scan across the imaged region of the ocular fundus 12. The optical system 30 may further comprise additional optical components, such as optical components for filtering, polarising, guiding and/or collimating the light from the light source 20, and a cylindrical lens or other means of generating a line of light in case the optical system 30 is a line-field system.

It should be noted that the mirror-based ophthalmic imaging instrument 100 described above has been given by way of an example only, and that an ophthalmic imaging instrument according to other example embodiments may employ a lens-based optical system instead of a mirror-based one of the kind described with reference to Figure 4, with one or more lenses arranged to light-guiding functions of mirrors 36 and 37.

Referring again to Figure 2, the drive mechanism 32 may, as in the present example embodiment, comprise a galvanometer 32-1 having a first drive shaft 32-2, and further comprise a second drive shaft 32-3 which is attached to the rotatable mirror 31 and rotationally coupled to the first drive shaft 32-2 such that the rotatable mirror 31 undergoes the predetermined rotational motion MR when driven by the galvanometer 32-1, rotating alternatively clockwise and anticlockwise about a rotational axis passing through the first focal point 37-1 of the spheroidal mirror 37 by the predetermined angle 2ϕ, as described above. The encoder wheel 33-1 is mounted on the second drive shaft 32-2 to rotate with the rotatable mirror 31, such that the axis of rotation of the rotatable mirror 31 coincides with the axis of rotation 33-4 of the encoder wheel 33-1. The rotational coupling 32- may take any suitable form, such as a universal joint, which would be advantageous in case the axis of rotation of the second drive shaft 32-3 needs to be inclined with respect to an axis of rotation of the first drive shaft 32-2. The mounting of the rotatable mirror 31 on a separate drive shaft which is rotationally coupled to the native drive shaft 32-2 of the galvanometer 32-1 may allow the galvanometer 32-1 to be situated more remotely from the first focal point 37-1 of the spheroidal mirror 37 than it might otherwise be, thus reducing or avoiding an encroachment of the galvanometer 32-1 into a space within the optical system 30 that is traversed by the beam of light L_{T} during the imaging of the eye 10 by the ophthalmic imaging instrument 100. Mounting the encoder wheel 33-1 on the second drive shaft 32-2, to which the rotatable mirror 31 is attached (rather than the native drive shaft 32-3 of the galvanometer 32-1), would enable a deviation from the expected rotational motion MR of the rotatable mirror 31 to be detected by the processor 40 in case the rotational coupling 32-5 fails while the galvanometer 32-1 continues to operate as normal, thus improving reliability. The provision of the rotational coupling 32-5 in the form of a universal joint or similar may further increase the flexibility with which the galvanometer 32-1 can be accommodated within the optical system 30.

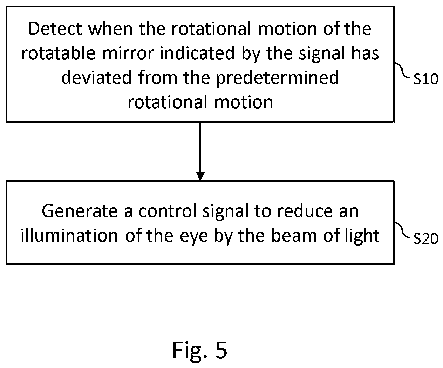

Operations performed by the processor 40 to generate the control signal Scto reduce an illumination of the eye 10 by the beam of light L_{T} upon detecting a deviation from an expected rotational motion MR of the rotatable mirror 31 will now be described with reference to the flow diagram shown in Figure 5.

In process S10 of Figure 5, the processor 40 detects when the rotational motion of the rotatable mirror 31 indicated by the signal S_{R} has deviated from the predetermined rotational motion MR. The processor 40 may perform this detection by repeatedly performing, for each time interval of a plurality of equal time intervals into which a period of the predetermined oscillatory rotational motion is divided, processes of: counting detections of markings on the encoder wheel 33-1 made by the detector 33-3 in the time interval; and detecting that the rotational motion of the rotatable mirror 31 has deviated from the predetermined rotational motion MR when the number of counted detections in the time interval is smaller than a threshold number of detections. Where the time intervals have a common duration, the number of marking detections in a time interval will tend to be higher where the time interval occurs in a part of the oscillatory cycle where the rotatable mirror 31 is rotating at a high angular velocity (i.e. where θ is close to zero) than in a part of the oscillatory cycle where the rotatable mirror 31 is rotating at a low angular velocity (i.e. where θ is close to -ϕ or +ϕ). In the present example embodiment, the duration of the time interval is set to be as short as possible whilst ensuring that at least the threshold number of detections (which may be a single detection or a small number of detections, for example 2 or 3 detections) is made in each of the time intervals as the rotatable mirror 31 undergoes a full cycle of the predetermined oscillatory rotational motion, in which the rotatable mirror 31 is rotating as is configured to rotate, with no failure in the drive mechanism 32 having occurred. Other schemes for detecting when the rotational motion of the rotatable mirror 31 indicated by the signal S_{R} has deviated from the predetermined rotational motion MR are also possible. For example, the threshold number of detections, with which the processor 40 compares the number of detections counted in a time interval, may be made dependent on the position of that time interval within the cycle of the oscillatory rotational motion, so that the threshold number of detections is higher where the time interval occurs while the rotatable mirror 31 has a relatively high angular velocity, than in the case where the time interval occurs while the rotatable mirror 31 has a relatively low angular velocity. This may allow the processor 40 to determine a departure from the expected oscillatory rotational motion sooner than in the case where the threshold number of detections is the same for all the time intervals.

In a variant of the present example embodiment, a similar advantage may be achieved by making a modification to the rotary encoder's hardware, rather than the functionality of the processor 40. More particularly, as illustrated schematically in Figure 3B, the markings 33-2' of the variant encoder wheel 33-1' may be provided at non-uniformly spaced angular positions about the axis of rotation 33-4 of the encoder wheel 33-1', which have angular separations therebetween that decrease towards each marking of the markings 33-2' which is last detected by the detector 33-3 before the encoder wheel 33-1' changes its direction of rotation when rotating with the rotatable mirror 31 alternatively clockwise and anticlockwise by the predetermined angle when the rotatable mirror 31 undergoes the predetermined oscillatory rotational motion. These two markings are referred to as "end markings" hereinafter. The angular separations between the markings 33-2' may progressively decrease towards each of the end markings such that the detector 33-3 detects the at least some of the markings 33-2' at a constant frequency when the encoder wheel 33-1' rotates alternatively clockwise and anticlockwise with the rotatable mirror 31. The end markings of the non-uniformly spaced markings 33-2' are labelled EM1 and EM2 in Figure 3B.

Referring again to Figure 5, in response to detecting that the rotational motion of the rotatable mirror 31 has deviated from the predetermined rotational motion MR in process S10, the processor 40 generates a control signal Sc in process S20, to reduce an illumination of the eye 10 by the beam of light L_{T}. The generated control signal Sc may, as in the present example embodiment, control the power supply 70 to reduce power supplied to the light source 20 to a low level which is safe for the eye 10 even when the beam of light L_{T} is incident on the eye 10 whilst not being scanned across the eye 10, or to zero power so that the light source 20 is turned off. Where the ophthalmic imaging instrument 100 is provided with the shutter 80 as described above, the generated control signal Sc may control the shutter 80 to close, thereby preventing the beam of light L_{T} from reaching the eye 10. For improved reliability, the processor 40 may respond to the detection of the deviation by both controlling the power supply 70 to reduce the power supplied to the light source 20 and controlling the shutter 80 to close. In other example embodiments, the generated control signal Sc may control an optical element of the optical system 30, such as a rotatable mirror, to deflect the beam of light L_{T} away from the eye 10.

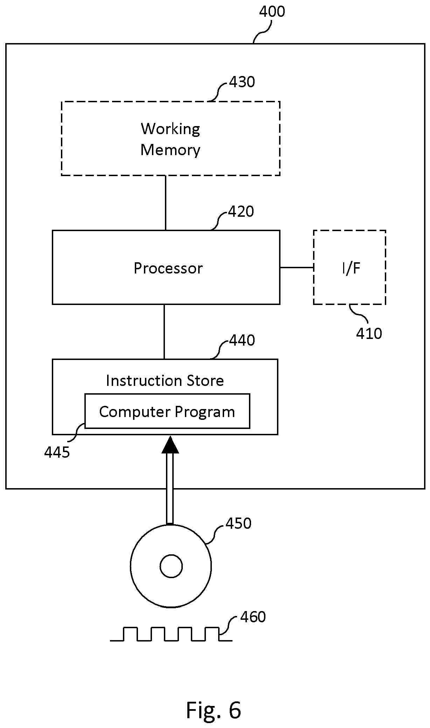

The processor 40 may be provided in any suitable form, for example as a processor 420 of a programmable signal processing hardware 400 of the kind illustrated schematically in Figure 6. The programmable signal processing hardware 400 comprises a communication interface (I/F) 410, for receiving the signal S_{R} from the rotary encoder 33, and outputting the control signal S_{C} to the power supply 70 and/or the shutter 80, for example. The I/F 410 may also send control signals to other components of the ophthalmic imaging instrument 100 for controlling it to illuminate the ocular fundus 12 and acquire images by detecting light L_{R} from the illuminated region of the ocular fundus 12. The signal processing hardware 400 further comprises a processor 420 (e.g. a Central Processing Unit, CPU, and/or a Graphics Processing Unit, GPU), a working memory 430 (e.g. a random-access memory) and an instruction store 440 storing a computer program 445 comprising the computer-readable instructions which, when executed by the processor 420, cause the processor 420 to perform various functions of the processor 40 described herein.

The working memory 430 stores information used by the processor 420 during execution of the computer program 445. The instruction store 440 may comprise a ROM (e.g. in the form of an electrically erasable programmable read-only memory (EEPROM) or flash memory) which is pre-loaded with the computer-readable instructions. Alternatively, the instruction store 440 may comprise a RAM or similar type of memory, and the computer-readable instructions of the computer program 445 can be input thereto from a computer program product, such as a non-transitory, computer-readable storage medium 450 in the form of a CD-ROM, DVDROM, etc. or a computer-readable signal 460 carrying the computer-readable instructions. In any case, the computer program 445, when executed by the processor 420, causes the processor 420 to perform the functions of the processor 40 as described herein. In other words, the processor 40 of the present example embodiment may comprise a computer processor 420 and a memory 440 storing computer-readable instructions which, when executed by the computer processor 420, cause the computer processor 420 to perform the functions of the processor 40 as described herein.

It should be noted, however, that the processor 40 may alternatively be implemented in non-programmable hardware, such as an ASIC, an FPGA or other integrated circuit dedicated to performing the functions of the processor 40 described herein, or a combination of such non-programmable hardware and programmable hardware as described above with reference to Figure 6.

As well as allowing a deviation from an expected rotational motion MR of the rotatable mirror 31 to be detected, so that appropriate action can be taken to prevent a portion of the eye 10 from receiving an unacceptably high dose of radiation from the light source 20, the output signal S_{R} of the rotary encoder 33 may also be used to tag or label image data acquired by the ophthalmic imaging instrument 100 from different scan locations on the fundus 12 with respective indications of those scan locations. Accordingly, the processor may, as in the present example embodiment, be arranged to generate a respective indication of a respective scan position of the beam of light L_{T} on the fundus 12 based on a detection by the detector 33-3 of each marking of the encoder wheel 33-1, and store the respective indication in association with respective image data acquired by the ophthalmic imaging instrument 100 at the respective scan position. The indications of the scan location may be obtained by calibration, using a test imaging target provided with a set of measurement markings, for example.

### [Example Embodiment 2]

In the first example embodiment, where the spheroidal mirror 37 (as an example of a curved mirror having an axis of circular symmetry) is employed to transfer the scanned beam of light L_{T} to the pupil 14 from a point on the rotatable mirror 31 which is at the first focal point 37-1 of the spheroidal mirror 37, and the axis of rotation of the rotatable mirror 31 is aligned with the axis of circular symmetry of the spheroidal mirror 37, the circular symmetry of the spheroidal mirror 37 gives rise to a linearity in the variation of the angle of rotation of the rotatable mirror 31 with an angle of incidence of the beam of light L_{T} on a pupillary plane of the eye 10 at the second focal point 37-2 of the spheroidal mirror 37, and therefore with the distance on the funds 12 traversed by the incident beam of light L_{T}. This allows the output signal S_{R} of the rotary encoder 33 to be used to tag or label image data acquired by the ophthalmic imaging instrument 100 from different scan locations on the fundus 12 with respective indications of those scan locations, as described above.

However, in an example embodiment like the present, where a curved mirror, which does not have an axis of circular symmetry aligned with the axis of rotation of the rotatable mirror 31, is provided instead of the spheroidal mirror 37, the linearity between the angle of rotation of the rotatable mirror 31 and the angle of incidence of the beam of light L_{T} on the pupillary plane (discussed above) may not occur. In this case, although the output of the rotary encoder 33 could be remapped by the processor 40 to correctly tag or label image data acquired by the ophthalmic imaging instrument from different scan locations on the fundus 12 with respective indications of those scan locations, this would undesirably increase the complexity of the data processing. An alternative approach to achieving this linearisation, specifically by an adaptation of the rotary encoder 33, will now be described with reference to Figures 3C, 3D, 3E, 7 and 8.

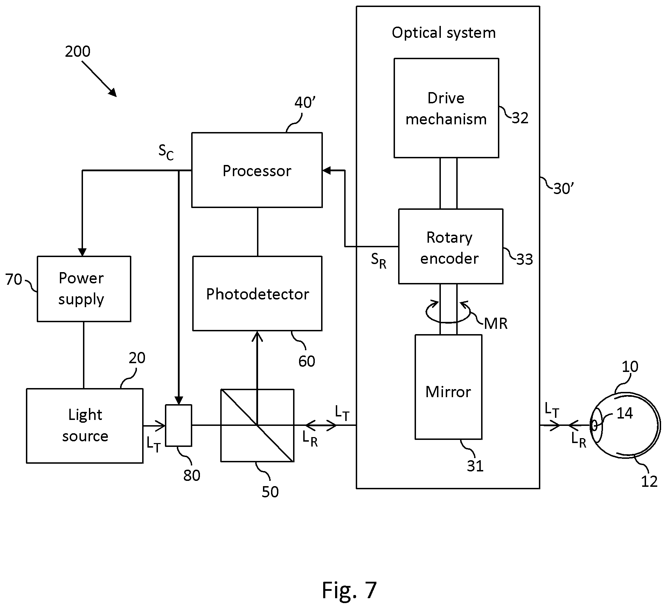

Figure 7 is a schematic illustration of an ophthalmic imaging instrument 200 of the second example embodiment, which differs from the ophthalmic imaging instrument 100 of the first example embodiment only by the configurations of the rotary encoders 33 and 38, the optical systems 30 and 30', and the processor 40 and 40' of these example embodiments, with all other components being the same. The rotary encoder 38, the optical system 30' and the processor 40' of the present example embodiment will now be described.

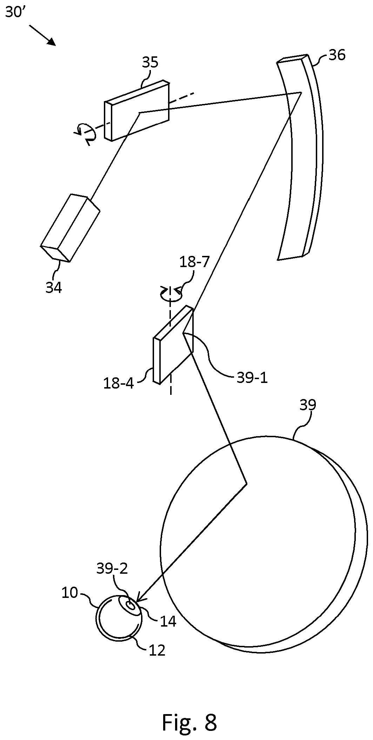

As illustrated in Figure 8, the optical system 30' of the present example embodiment comprises a curved mirror 39, via which the rotatable mirror 31 is arranged to scan the beam of light L_{T} across the fundus 12. The curved mirror 39 may be an ellipsoidal mirror having three different semi-axes, for example. The curved mirror 39 has a first focal point 39-1 and a second focal point 39-2. The rotatable mirror 31 is provided at the first focal point 39-1, so as to reflect the beam of light L_{T} at the first focal point 39-1, and the pupil 14 of the eye 10 is positioned at the second focal point 39-2 during the imaging of the fundus 12. The curvature of the curved mirror 39 is such that an angle of incidence of the beam of light L_{T} on a pupillary plane of the eye 10 at the second focal point 39-2 varies non-linearly with the angle of rotation of the rotatable mirror 31 about its axis of rotation. Accordingly, a given incremental rotation of the rotatable mirror 31 will generally cause the beam of light L_{T} to traverse a section of the fundus 12 whose length depends on the angle of rotation of the rotatable mirror 21 from which the incremental rotation is made.

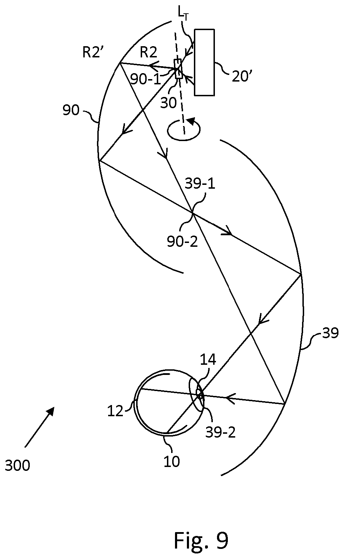

It should be noted that, although the rotatable mirror 31 is arranged to reflect the beam of light L_{T} at the first focal point 39-1 of the curved mirror 39, such that the beam of light L_{T} is scanned across the curved mirror 39 as the rotatable mirror 31 rotates, the optical system 30' may be arranged such that the rotatable mirror 31 scans the beam L_{T} via the first focal point 39-1 in another way. Figure 9 is a schematic illustration of a line-scan UWF ophthalmic imaging instrument 300 according to a variant of the second example embodiment which comprises, in addition to the curved mirror 39, a second curved (e.g. ellipsoidal) mirror 90, via which the rotatable mirror 31 is arranged to scan a beam of light L_{T}' in the form of a line of light across the fundus 12. In this case, the beam of light L_{T}' has a projection onto a plane normal to its direction of propagation (i.e. a cross-section) which is a line, and may be generated from a beam of light having a cross-section of a spot (as generated by the light source 20) using a cylindrical lens, or may be generated using another optical arrangement for generating line-field illumination known to those versed in the art (e.g. a back-lit slit aperture). As illustrated in Figure 9, the second curved mirror 90 has a first focal point 90-1 and a second focal point 90-2, with the rotatable mirror 31 being arranged to reflect the beam of light L_{T}' at the first focal point 90-1 of the second curved mirror 90. The second focal point 90-2 of the second curved mirror 90 coincides with the first focal point 39-1 of the curved mirror 39.

The light source 20', which generates the line-field illumination L_{T}', is shown in Figure 9, and this is provided instead of the light source 20, the first scanning element 35 and the first curved mirror 36 of the first example embodiment. The line-scan UWF ophthalmic imaging instrument 300 includes the remaining components of the UWF ophthalmic imaging instrument 200, although these are not shown in Figure 9 to more clearly illustrate the different scan transfer arrangement comprising the two curved (e.g. ellipsoidal) mirrors 39 and 90. The line-scan UWF ophthalmic imaging instrument 300 also has a beam splitter to direct the return light from the fundus 12 to a photodetector for detecting the return line-field illumination, although this has similarly been omitted from Figure 9 for clarity.

It is noted that the UWF ophthalmic imaging instrument 100 of the first example embodiment may be similarly adapted to include the arrangement of curved mirrors as described above with reference to Figure 9, to provide an alternative way of scanning the beam of light L_{T} across the spheroidal mirror 37 via the first focal point 37-1 of the spheroidal mirror 37.

As illustrated in Figure 3C, in the present example embodiment, the markings 38-2 of the encoder wheel 38-1 are provided at non-uniformly spaced angular positions about the axis of rotation 33-4 of the encoder wheel 38-1. However, the angular distribution of the markings 38-2 in Figure 3C is different from the angular distribution of the markings 33-2 and 33-2' in Figures 3A and 3B, respectively, and is such that, as the beam of light L_{T} is scanned via the curved mirror 39, the same change in the angle of incidence on the pupillary plane occurs as the rotatable mirror 31 rotates between detections by the detector 33-3 of different adjacent markings 38-2 of the encoder wheel 38-1. In other words, the respective rotation of the rotatable mirror 31 that occurs between detections by the detector 33-3 of the markings in each pair of adjacent markings 38-2 of the encoder wheel 38-1 is the same. Accordingly, each detection of a marking in a series of detections of the markings 38-2 made by the detector 33-3 of the rotary encoder 38 corresponds to the same change in the angle of incidence on the pupillary plane having occurred since the previous detection was made. The spacings between the markings 38-2 may be calculated using the results of a simulation of ray propagation in the optical system 30' that may be performed using any well-known ray tracing software package that is suitable for this purpose, for example.

In the present example embodiment, the processor 40' is arranged to generate a respective indication of a respective scan position of the beam of light L_{T} on the fundus 12 based on a detection by the detector 33-3 of each marking of the encoder wheel 38-1, and associate the respective indication with respective image data acquired by the ophthalmic imaging instrument 200 at the respective scan position. The processor 40' is further configured to detect when the rotational motion of the rotatable mirror 31 indicated by the signal S_{R} has deviated from the predetermined rotational motion MR, and to generate the control signal S_{C} to reduce an illumination of the eye 10 by the beam of light L_{T} in response to making such detection, as described above in the context of the first example embodiment. However, as noted below, this further configuration of the processor 40 is optional and may not be present in some example embodiments.

To allow the processor 40' to detect the deviation from the predetermined rotational motion MR more rapidly, the rotary encoder 38 of the present example embodiment may be modified to comprise, in addition to the set of markings 38-2 described above, the set of markings 33-2 of the first example embodiment, or the set of markings 33-2' of the variant of the first example embodiment described above with reference to Figure 3B.

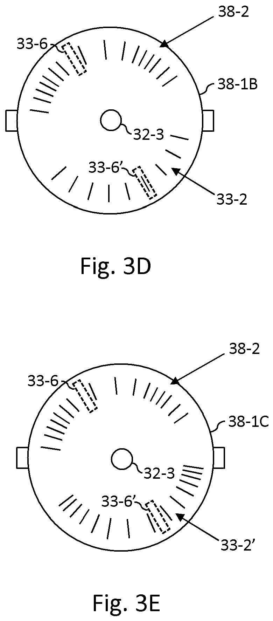

For example, the markings 38-2 and 33-2 may be provided on a single encoder wheel 38-1B, as illustrated schematically in Figure 3D. Figure 3D also shows the position of a second reading region 33-6' of the detector 33-3, in which the detector 33-3 is able to detect the presence of a marking of the markings 33-2. The position of the second reading region 33-6' does not change as the encoder wheel 38-1B rotates. As another example, the markings 38-2 and 33-2' may be provided on a single encoder wheel 38-1C, as illustrated schematically in Figure 3E. Alternatively, the rotary encoder 38 may comprise the encoder wheel 33-1 having markings 33-2, and the encoder wheel 38-1 having markings 38-2, with the two encoder wheels being mounted coaxially on the drive shaft 32-3. As a further alternative, the rotary encoder 38 may comprise the encoder wheel 33-1' having markings 33-2', and the encoder wheel 38-1 having markings 38-2, with the two encoder wheels again being mounted coaxially on the drive shaft 32-3. It is noted that the relative arrangements of the markings 38-2 and 33-2 on encoder wheel 38-1B, and the relative arrangements of the markings 38-2 and 33-2' on encoder wheel 38-1C, are given by way of an example only, and that other arrangements of the two sets of markings on a single encoder wheel are possible, provided that the detector 33 is adapted (for example to have more than one photosensor, if necessary) to detect both sets of markings.

In some variants of the second example embodiment, wherein the rotary encoder 38 includes the markings 38-2 and processor 40' to achieve the linearisation described above, the drive mechanism 32, which is arranged to drive the rotatable mirror 31 to undergo the predetermined rotational motion MR to scan the beam of light L_{T} across the ocular fundus 12, may be omitted, and the processor 40' need not be arranged to perform the processes of: monitoring detections of the markings 38-2 by the detector 33-3; detecting, from the monitored detections, when the rotational motion of the rotatable mirror 31 has deviated from the predetermined rotational motion MR; and in response to detecting that the rotational motion of the rotatable mirror 31 has deviated from the predetermined rotational motion MR, generating a control signal Sc to reduce an illumination of the eye 10 by the beam of light L_{T}. These variants are summarised in the following numbered clauses E1 to E6.

E1. An ultra-widefield ophthalmic imaging instrument for imaging a fundus (12) of an eye (10) of a subject, comprising:
a light source (20) arranged to emit a beam of light (L_{T}) for imaging the fundus (12);
an optical system (30') comprising:
   a rotatable mirror (31) arranged to scan the beam of light (L_{T}) across the fundus (12); and
   a curved mirror (39) via which the rotatable mirror (31) is arranged to scan the beam of light (L_{T}) across the fundus (12), the curved mirror (39) having a first focal point (39-1) and a second focal point (39-2), wherein
   the rotatable mirror (31) is arranged to scan the beam of light (L_{T}) across the curved mirror (39) via the first focal point (39-1), and a pupil (14) of the eye (10) is positioned at the second focal point (39-2) during the imaging of the fundus (12), and
   the curved mirror (39) has a curvature such that an angle of incidence of the beam of light (L_{T}) on a pupillary plane of the eye (10) at the second focal point (39-2) varies non-linearly with an angle of rotation of the rotatable mirror (31);
a rotary encoder (33) coupled to the rotatable mirror (31) so as to generate a signal (S_{R}) indicative of a rotational motion (MR) of the rotatable mirror (31), the rotary encoder comprising:
   an encoder wheel (38-1) attached to the rotatable mirror (31) so as to rotate with the rotatable mirror (31), the encoder wheel (38-1) having markings (38-2) for detecting a rotation of the encoder wheel (38-1); and
   a detector (33-3) arranged to detect the markings (38-2) on the encoder wheel (38-1) as the encoder wheel (38-1) rotates,
   wherein the markings (38-2) are provided at non-uniformly spaced angular positions about an axis of rotation of the encoder wheel (38-1) such that, as the beam of light (L_{T}) is scanned via the curved mirror (39), a common change in the angle of incidence occurs as the rotatable mirror (31) rotates between detections by the detector (33-3) of different adjacent markings of the markings (38-2); and
a processor (40') is arranged to generate a respective indication of a respective scan position of the beam of light (L_{T}) on the fundus (12) based on a detection by the detector (33-3) of each of the markings (38-2), and associate the respective indication with respective image data acquired by the ophthalmic imaging instrument (100) at the respective scan position.

E2. The ophthalmic imaging instrument according to E1, wherein
the optical system (30') further comprises a drive mechanism (32) arranged to drive the rotatable mirror (31) to undergo a predetermined rotational motion (MR) to scan the beam of light (L_{T}) across the fundus (12), and
the processor (40') is further arranged to:
   monitor detections of the markings (38-2) by the detector (33-3);
   detect, from the monitored detections, when the rotational motion of the rotatable mirror (31) has deviated from the predetermined rotational motion (MR); and
   in response to detecting that the rotational motion of the rotatable mirror (31) has deviated from the predetermined rotational motion (MR), generate a control signal (Sc) to reduce an illumination of the eye (10) by the beam of light (L_{T}).

E3. The ophthalmic imaging instrument according to E2, wherein the processor (40') is arranged to detect when the rotational motion of the rotatable mirror (31) has deviated from the predetermined rotational motion (MR) by repeatedly preforming, for each time interval of a plurality of time intervals, processes of:
counting detections by the detector (33-3) in the time interval; and
detecting that the rotational motion of the rotatable mirror (31) has deviated from the predetermined rotational motion (MR) when the counted detections in the time interval are fewer than a threshold number of detections.

E4. The ophthalmic imaging instrument according to E2 or E3, wherein
the drive mechanism (32) comprises:
   a galvanometer (32-1) having a first drive shaft (32-2); and
   a second drive shaft (32-3) which is attached to the rotatable mirror (31) and rotationally coupled to the first drive shaft (32-2) such that the rotatable mirror (31) undergoes the predetermined rotational motion (MR) when driven by the galvanometer (32-1), and
the encoder wheel (38-1) is mounted on the second drive shaft (32-2) to rotate with the rotatable mirror (31).

E5. The ophthalmic imaging instrument according to any of E2 to E4, further comprising at least one of:
a power supply (70) arranged to supply power to the light source (20), wherein the generated control signal (S_{C}) is arranged to control the power supply (70) to reduce power supplied to the light source (20); or
a shutter (80) operable to prevent the beam of light (L_{T}) from reaching the eye (10), wherein the generated control signal (Sc) is arranged to control the shutter (80) to prevent the beam of light (L_{T}) from reaching the eye (10).

E6. The ophthalmic imaging instrument according to any of E2 to E5, wherein the rotatable mirror (31) is arranged to reflect the beam of light (L_{T}) at the first focal point (39-1) towards the curved mirror (39).

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the functions of the processor 40, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein.

Some or all of the functionality of the processor 40 may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. An ophthalmic imaging instrument for imaging an eye (10) of a subject, comprising:
a light source (20) arranged to emit a beam of light (L_{T}) for imaging the eye (10);
an optical system (30; 30') comprising a rotatable mirror (31) and a drive mechanism (32) arranged to drive the rotatable mirror (31) to undergo a predetermined rotational motion (MR) to scan the beam of light (L_{T}) across the eye (10);
a rotary encoder (33) coupled to the rotatable mirror (31) so as to generate a signal (S_{R}) indicative of a rotational motion of the rotatable mirror (31); and
a processor (40; 40') arranged to:
detect when the rotational motion of the rotatable mirror (31) indicated by the signal (S_{R}) has deviated from the predetermined rotational motion (MR); and
in response to detecting that the rotational motion of the rotatable mirror (31) has deviated from the predetermined rotational motion (MR), generate a control signal (S_{C}) to reduce an illumination of the eye (10) by the beam of light (L_{T}).

2. The ophthalmic imaging instrument according to Claim 1, wherein
the rotary encoder (33; 38A) comprises:
an encoder wheel (33-1; 33-1'; 38-1) attached to the rotatable mirror (31) so as to rotate with the rotatable mirror (31), the encoder wheel (33-1; 33-1'; 38-1) having markings (33-2; 33-2'; 38-2) for detecting a rotation of the encoder wheel (33-1; 33-1'; 38-1); and
a detector (33-3) arranged to detect the markings (33-2; 33-2'; 38-2) on the encoder wheel (33-1; 33-1'; 38-1) as the encoder wheel (33-1; 33-1'; 38-1) rotates; and
the processor (40; 40') is arranged to:
monitor detections of the markings (33-2; 33-2'; 38-2) by the detector (33-3); and
detect, from the monitored detections, when the rotational motion of the rotatable mirror (31) has deviated from the predetermined rotational motion (MR).

3. The ophthalmic imaging instrument according to Claim 2, wherein the markings (33-2) of the encoder wheel (33-1) are provided at regularly spaced angular positions about an axis of rotation (33-4) of the encoder wheel (33-1).

4. The ophthalmic imaging instrument according to Claim 3, wherein
the ophthalmic imaging instrument is an ultra-widefield ophthalmic imaging instrument for imaging a fundus (12) of the eye (10), wherein the optical system (30) further comprises a curved mirror (37) via which the rotatable mirror (31) is arranged to scan the beam of light (L_{T}) across the fundus (12), the curved mirror (37) having an axis of circular symmetry, a first focal point (37-1) and a second focal point (37-2), wherein the rotatable mirror (31) is arranged to scan the beam of light (L_{T}) across the curved mirror (37) via the first focal point (37-1) and has an axis of rotation aligned with the axis of circular symmetry of the curved mirror (37), and a pupil (14) of the eye (10) is positioned at the second focal point (37-2) during the imaging of the fundus (12), and
the processor (40) is further arranged to generate a respective indication of a respective scan position of the beam of light (L_{T}) on the fundus (12) based on a detection by the detector (33-3) of each marking of the encoder wheel (33-1), and associate the respective indication with respective image data acquired by the ophthalmic imaging instrument at the respective scan position.

5. The ophthalmic imaging instrument according to Claim 2, wherein
the predetermined rotational motion is a predetermined oscillatory rotational motion, wherein the rotatable mirror (31) rotates alternatively clockwise and anticlockwise by a predetermined angle, and
the markings (33-2') of the encoder wheel (33-1') are provided at non-uniformly spaced angular positions about an axis of rotation (33-4) of the encoder wheel (33-1') that have angular separations therebetween which decrease towards each marking (EM1; EM2) of the markings (33-2') which is last detected by the detector (33-3) before the encoder wheel (33-1') changes its direction of rotation when rotating with the rotatable mirror (31) alternatively clockwise and anticlockwise by the predetermined angle.

6. The ophthalmic imaging instrument according to Claim 5, wherein at least some of the markings (33-2') of the encoder wheel (33-1') are provided at non-uniformly spaced angular positions about the axis of rotation (33-4) of the encoder wheel (33-1') such that the detector (33-3) detects the at least some of the markings (33-2) at a constant frequency when the encoder wheel (33-1) rotates alternatively clockwise and anticlockwise with the rotatable mirror (31) when undergoing the predetermined oscillatory rotational motion.

7. The ophthalmic imaging instrument according to Claim 2, wherein
the ophthalmic imaging instrument is an ultra-widefield ophthalmic imaging instrument for imaging a fundus (12) of the eye (10), wherein the optical system (30') further comprises a curved mirror (39) via which the rotatable mirror (31) is arranged to scan the beam of light (L_{T}) across the fundus (12), the curved mirror (39) having a first focal point (39-1) and a second focal point (39-2), wherein the rotatable mirror (31) is arranged to scan the beam of light (L_{T}) across the curved mirror (39) via the first focal point (39-1), and a pupil (14) of the eye (10) is positioned at the second focal point (39-2) during the imaging of the fundus (12),
the curved mirror (39) has a curvature such that an angle of incidence of the beam of light (L_{T}) on a pupillary plane of the eye (10) at the second focal point (39-2) varies non-linearly with an angle of rotation of the rotatable mirror (31),
the markings (38-2) of the encoder wheel (38-1) are provided at non-uniformly spaced angular positions about an axis of rotation (33-4) of the encoder wheel (38-1) such that, as the beam of light (L_{T}) is scanned via the curved mirror (39), a common change in the angle of incidence occurs as the rotatable mirror (31) rotates between detections by the detector (33-3) of different adjacent markings (38-2) of the encoder wheel (38-1), and
the processor (40') is further arranged to generate a respective indication of a respective scan position of the beam of light (L_{T}) on the fundus (12) based on a detection by the detector (33-3) of each marking of the encoder wheel (38-1), and associate the respective indication with respective image data acquired by the ophthalmic imaging instrument (200) at the respective scan position.

8. The ophthalmic imaging instrument according to any of Claims 2, 3, 5 and 6, wherein
the ophthalmic imaging instrument (200) is an ultra-widefield ophthalmic imaging instrument for imaging a fundus (12) of the eye (10), wherein the optical system (30') further comprises a curved mirror (39) via which the rotatable mirror (31) is arranged to scan the beam of light (L_{T}) across the fundus (12), the curved mirror (39) having a first focal point (39-1) and a second focal point (39-2), wherein the rotatable mirror (31) is arranged to scan the beam of light (L_{T}) across the curved mirror (39) via the first focal point (39-1), and a pupil (14) of the eye (10) is positioned at the second focal point (39-2) during the imaging of the fundus (12),
the curved mirror (39) has a curvature such that an angle of incidence of the beam of light (L_{T}) on a pupillary plane of the eye (10) at the second focal point (39-2) varies non-linearly with an angle of rotation of the rotatable mirror (31),
the rotary encoder (38) further comprises second markings (38-2) that are detectable by the detector (33-3) as the encoder wheel (38-1; 38-1B; 38-1C) rotates, the second markings (38-2) being provided at non-uniformly spaced angular positions about an axis of rotation of the second markings (38-2) such that, as the beam of light (L_{T}) is scanned via the curved mirror (39), a common change in the angle of incidence occurs as the rotatable mirror (31) rotates between detections by the detector (33-3) of different adjacent markings of the second markings (38-2), and
the processor (40') is further arranged to generate a respective indication of a respective scan position of the beam of light (L_{T}) on the fundus (12) based on a detection by the detector (33-3) of each second marking, and associate the respective indication with respective image data acquired by the ophthalmic imaging instrument at the respective scan position.

9. The ophthalmic imaging instrument according to Claim 8, wherein the second markings (38-2) are provided on the encoder wheel (38-1B; 38-1C).

10. The ophthalmic imaging instrument according to Claim 8, wherein the rotary encoder (38) further comprises a second encoder wheel (38-1) attached to the rotatable mirror (31) so as to rotate with the rotatable mirror (31), the second markings (38-2) being provided on the second encoder wheel (38-1), and the detector (33-3) is arranged to detect the second markings (38-2) on the second encoder wheel (38-1) as the second encoder wheel (38-1) rotates.

11. The ophthalmic imaging instrument according to any of Claims 2 to 10, wherein the processor (40; 40') is arranged to detect when the rotational motion of the rotatable mirror (31) has deviated from the predetermined rotational motion (MR) by repeatedly preforming, for each time interval of a plurality of time intervals, processes of:
counting detections by the detector (33-3) in the time interval; and
detecting that the rotational motion of the rotatable mirror (31) has deviated from the predetermined rotational motion (MR) when the counted detections in the time interval are fewer than a threshold number of detections.

12. The ophthalmic imaging instrument according to any of Claims 2 to 11, wherein
the drive mechanism (32) comprises:
a galvanometer (32-1) having a first drive shaft (32-2); and
a second drive shaft (32-3) which is attached to the rotatable mirror (31) and rotationally coupled to the first drive shaft (32-2) such that the rotatable mirror (31) undergoes the predetermined rotational motion (MR) when driven by the galvanometer (32-1), and
the encoder wheel (33-1; 38-1; 381B; 38-1C) is mounted on the second drive shaft (32-2) to rotate with the rotatable mirror (31).

13. The ophthalmic imaging instrument according to any preceding claim, further comprising at least one of:
a power supply (70) arranged to supply power to the light source (20), wherein the generated control signal (S_{C}) is arranged to control the power supply (70) to reduce power supplied to the light source (20); or
a shutter (80) operable to prevent the beam of light (L_{T}) from reaching the eye (10), wherein the generated control signal (Sc) is arranged to control the shutter (80) to prevent the beam of light (L_{T}) from reaching the eye (10).

14. A computer-implemented method of generating a control signal (Sc) for controlling an ophthalmic imaging instrument for imaging an eye (10) of a subject, the ophthalmic imaging instrument comprising:
a light source (20) arranged to emit a beam of light (L_{T}) for imaging the eye (10);
an optical system (30) comprising a rotatable mirror (31) and a drive mechanism (32) arranged to drive the rotatable mirror (31) to undergo a predetermined rotational motion (MR) to scan the beam of light (L_{T}) across the eye (10); and
a rotary encoder (33) coupled to the rotatable mirror (31) so as to generate a signal (S_{R}) indicative of a rotational motion of the rotatable mirror (31),
the method comprising:
detecting (S10) when the rotational motion of the rotatable mirror (31) indicated by the signal (S_{R}) has deviated from the predetermined rotational motion (MR); and
in response to detecting that the rotational motion of the rotatable mirror (31) has deviated from the predetermined rotational motion (MR), generating (S20) a control signal (S_{C}) to reduce an illumination of the eye (10) by the beam of light (L_{T}).

15. A computer program (445) comprising computer-readable instructions that, when executed by at least one processor (420), cause the at least one processor (420) to perform the method according to Claim 14.
